# EUROPEAN PATENT APPLICATION

(11) **EP 2 713 161 A1**
(43) Date of publication of application: **02.04.2014**
(21) Application number: 12186303.9
(22) Date of filing: 27.09.2012
(51) Int. Cl.: G01N 33/53

(54) **Method of detection of Schmallenberg virus (SBV) and kit**

(71) Applicant: IDEXX LABORATORIES, INC., Westbrook, Maine 04092 (US)
(72) Inventor: Schaller, Alain, 1630 Bulle (CH); Sénéchal, Yann, 1740 Neyruz (CH); Pun, San, 4129 Riehen (CH); Schelp, Christian, 3110 Münsingen (CH); Krah III, Eugene Regis, Yarmouth, ME 04096 (US); Siefring, Ann Elizabeth, Gorham, ME 04038 (US)
(74) Representative: Helbig, Christian

(57) **Abstract**

The invention relates to Orthobunyavirus, in particular to Schmallenberg Virus (SBV), and a diagnostic test and kit for the detection of SBV infection and of a Simbu serogroup test.

SEQUENCE LISTING

<110>  IDEXX Laboratories Inc.

<120>  SBV disgnostic test and kit

<130>  IX20155EP

<160>  5

<170>  PatentIn version 3.5

<210>  1
<211>  233
<212>  PRT
<213>  SBV Schmallenberg Virus cattle - nucleocapsid protein full length

<400>  1

Met Ser Ser Gln Phe Ile Phe Glu Asp Val Pro Gln Arg Asn Ala Ala
1               5                   10                  15

Thr Phe Asn Pro Glu Val Gly Tyr Val Ala Phe Ile Gly Lys Tyr Gly
            20                  25                  30

Gln Gln Leu Asn Phe Gly Val Ala Arg Val Phe Phe Leu Asn Gln Lys
        35                  40                  45

Lys Ala Lys Met Val Leu His Lys Thr Ala Gln Pro Ser Val Asp Leu
    50                  55                  60

Thr Phe Gly Gly Val Lys Phe Thr Val Val Asn Asn His Phe Pro Gln
65                  70                  75                  80

Tyr Val Ser Asn Pro Val Pro Asp Asn Ala Ile Thr Leu His Arg Met
                85                  90                  95

Ser Gly Tyr Leu Ala Arg Trp Ile Ala Asp Thr Cys Lys Ala Ser Val
            100                 105                 110

Leu Lys Leu Ala Glu Ala Ser Ala Gln Ile Val Met Pro Leu Ala Glu
        115                 120                 125

Val Lys Gly Cys Thr Trp Ala Asp Gly Tyr Thr Met Tyr Leu Gly Phe
    130                 135                 140

Ala Pro Gly Ala Glu Met Phe Leu Asp Ala Phe Asp Phe Tyr Pro Leu
145                 150                 155                 160

Val Ile Glu Met His Arg Val Leu Lys Asp Asn Met Asp Val Asn Phe
                165                 170                 175

Met Lys Lys Val Leu Arg Gln Arg Tyr Gly Thr Met Thr Ala Glu Glu
            180                 185                 190

Trp Met Thr Gln Lys Ile Thr Glu Ile Lys Ala Ala Phe Asn Ser Val
      195                 200                 205

Gly Gln Leu Ala Trp Ala Lys Ser Gly Phe Ser Pro Ala Ala Arg Thr
    210                 215                 220

Phe Leu Gln Gln Phe Gly Ile Asn Ile
225                 230

<210>  2
<211>  253
<212>  PRT
<213>  SBV Schmallenberg Virus cattle - nucleocapsid protein full length his
tag

<400>  2

Met Gly Ser Ser His His His His His His Ser Ser Gly Leu Val Pro
1               5                   10                  15

Arg Gly Ser His Met Ser Ser Gln Phe Ile Phe Glu Asp Val Pro Gln
            20                  25                  30

Arg Asn Ala Ala Thr Phe Asn Pro Glu Val Gly Tyr Val Ala Phe Ile
        35                  40                  45

Gly Lys Tyr Gly Gln Gln Leu Asn Phe Gly Val Ala Arg Val Phe Phe
    50                  55                  60

Leu Asn Gln Lys Lys Ala Lys Met Val Leu His Lys Thr Ala Gln Pro
65                  70                  75                  80

Ser Val Asp Leu Thr Phe Gly Gly Val Lys Phe Thr Val Val Asn Asn
                85                  90                  95

His Phe Pro Gln Tyr Val Ser Asn Pro Val Pro Asp Asn Ala Ile Thr
            100                 105                 110

Leu His Arg Met Ser Gly Tyr Leu Ala Arg Trp Ile Ala Asp Thr Cys
        115                 120                 125

Lys Ala Ser Val Leu Lys Leu Ala Glu Ala Ser Ala Gln Ile Val Met
    130                 135                 140

Pro Leu Ala Glu Val Lys Gly Cys Thr Trp Ala Asp Gly Tyr Thr Met
145                 150                 155                 160

Tyr Leu Gly Phe Ala Pro Gly Ala Glu Met Phe Leu Asp Ala Phe Asp
                165                 170                 175

Phe Tyr Pro Leu Val Ile Glu Met His Arg Val Leu Lys Asp Asn Met
            180                 185                 190

Asp Val Asn Phe Met Lys Lys Val Leu Arg Gln Arg Tyr Gly Thr Met
        195                 200                 205

Thr Ala Glu Glu Trp Met Thr Gln Lys Ile Thr Glu Ile Lys Ala Ala
    210                 215                 220

Phe Asn Ser Val Gly Gln Leu Ala Trp Ala Lys Ser Gly Phe Ser Pro
225                 230                 235                 240

Ala Ala Arg Thr Phe Leu Gln Gln Phe Gly Ile Asn Ile
                245                 250

<210>  3
<211>  762
<212>  DNA
<213>  SBV Schmallenberg Virus cattle - nucleocapsid DNA in pET28a vector

<400>  3
atgggcagca gccatcatca tcatcatcac agcagcggcc tggtgccgcg cggcagccat      60

atgagcagcc agtttatctt tgaagatgtt ccgcagcgta atgcagcaac ctttaatccg     120

gaagttggtt atgttgcctt cattggtaaa tatggtcagc agctgaattt tggtgttgcc     180

cgtgtttttt ttctgaacca gaaaaaagcc aaaatggtgc tgcataaaac cgcacagccg     240

agcgttgatc tgacctttgg tggtgtgaaa tttaccgtgg tgaataatca ttttccgcag     300

tatgttagca atccggttcc ggataatgca attaccctgc atcgtatgag cggttatctg     360

gcacgttgga ttgcagatac ctgtaaagca agcgttctga aactggcaga agcaagcgca     420

cagattgtta tgccgctggc cgaagtgaaa ggttgtacct gggcagatgg ttataccatg     480

tatctgggtt ttgcaccggg tgcagaaatg tttctggatg cctttgattt ttatccgctg     540

gtgattgaaa tgcaccgtgt gctgaaagat aatatggatg tgaactttat gaaaaaagtg     600

ctgcgtcagc gttatggcac catgacagcc gaagaatgga tgacccagaa aattaccgaa     660

atcaaagcag cctttaatag cgttggtcag ctggcatggg caaaaagcgg ttttagtccg     720

gcagcacgta cctttctgca gcagtttggc attaatatct aa                        762

<210>  4
<211>  699
<212>  DNA
<213>  SBV Schmallenberg VIrus cattle - nucleocapsid protein DNA sequence
with consensus codons

<220>
<221>  misc_feature
<222>  (6)..(6)
<223>  n is a, c, g, or t

<220>
<221>  misc_feature
<222>  (9)..(9)
<223>  n is a, c, g, or t

<220>
<221>  misc_feature
<222>  (30)..(30)
<223>  n is a, c, g, or t

<220>
<221>  misc_feature
<222>  (33)..(33)
<223>  n is a, c, g, or t

<220>
<221>  misc_feature
<222>  (39)..(39)
<223>  n is a, c, g, or t

<220>
<221>  misc_feature
<222>  (45)..(45)
<223>  n is a, c, g, or t

<220>
<221>  misc_feature
<222>  (48)..(48)
<223>  n is a, c, g, or t

<220>
<221>  misc_feature
<222>  (51)..(51)
<223>  n is a, c, g, or t

<220>
<221>  misc_feature
<222>  (60)..(60)
<223>  n is a, c, g, or t

<220>
<221>  misc_feature
<222>  (66)..(66)
<223>  n is a, c, g, or t

<220>
<221>  misc_feature
<222>  (69)..(69)
<223>  n is a, c, g, or t

<220>
<221>  misc_feature
<222>  (75)..(75)
<223>  n is a, c, g, or t

<220>
<221>  misc_feature
<222>  (78)..(78)
<223>  n is a, c, g, or t

<220>
<221>  misc_feature
<222>  (87)..(87)
<223>  n is a, c, g, or t

<220>
<221>  misc_feature
<222>  (96)..(96)
<223>  n is a, c, g, or t

<220>
<221>  misc_feature
<222>  (105)..(105)
<223>  n is a, c, g, or t

<220>
<221>  misc_feature
<222>  (114)..(114)
<223>  n is a, c, g, or t

<220>
<221>  misc_feature
<222>  (117)..(117)
<223>  n is a, c, g, or t

<220>
<221>  misc_feature
<222>  (120)..(120)
<223>  n is a, c, g, or t

<220>
<221>  misc_feature
<222>  (123)..(123)
<223>  n is a, c, g, or t

<220>
<221>  misc_feature
<222>  (126)..(126)
<223>  n is a, c, g, or t

<220>
<221>  misc_feature
<222>  (135)..(135)
<223>  n is a, c, g, or t

<220>
<221>  misc_feature
<222>  (150)..(150)
<223>  n is a, c, g, or t

<220>
<221>  misc_feature
<222>  (159)..(159)
<223>  n is a, c, g, or t

<220>
<221>  misc_feature
<222>  (162)..(162)
<223>  n is a, c, g, or t

<220>
<221>  misc_feature
<222>  (171)..(171)
<223>  n is a, c, g, or t

<220>
<221>  misc_feature
<222>  (174)..(174)
<223>  n is a, c, g, or t

<220>
<221>  misc_feature
<222>  (180)..(180)
<223>  n is a, c, g, or t

<220>
<221>  misc_feature
<222>  (183)..(183)
<223>  n is a, c, g, or t

<220>
<221>  misc_feature
<222>  (186)..(186)
<223>  n is a, c, g, or t

<220>
<221>  misc_feature
<222>  (192)..(192)
<223>  n is a, c, g, or t

<220>
<221>  misc_feature
<222>  (195)..(195)
<223>  n is a, c, g, or t

<220>
<221>  misc_feature
<222>  (201)..(201)
<223>  n is a, c, g, or t

<220>
<221>  misc_feature
<222>  (204)..(204)
<223>  n is a, c, g, or t

<220>
<221>  misc_feature
<222>  (207)..(207)
<223>  n is a, c, g, or t

<220>
<221>  misc_feature
<222>  (216)..(216)
<223>  n is a, c, g, or t

<220>
<221>  misc_feature
<222>  (219)..(219)
<223>  n is a, c, g, or t

<220>
<221>  misc_feature
<222>  (222)..(222)
<223>  n is a, c, g, or t

<220>
<221>  misc_feature
<222>  (237)..(237)
<223>  n is a, c, g, or t

<220>
<221>  misc_feature
<222>  (246)..(246)
<223>  n is a, c, g, or t

<220>
<221>  misc_feature
<222>  (249)..(249)
<223>  n is a, c, g, or t

<220>
<221>  misc_feature
<222>  (255)..(255)
<223>  n is a, c, g, or t

<220>
<221>  misc_feature
<222>  (258)..(258)
<223>  n is a, c, g, or t

<220>
<221>  misc_feature
<222>  (261)..(261)
<223>  n is a, c, g, or t

<220>
<221>  misc_feature
<222>  (270)..(270)
<223>  n is a, c, g, or t

<220>
<221>  misc_feature
<222>  (276)..(276)
<223>  n is a, c, g, or t

<220>
<221>  misc_feature
<222>  (279)..(279)
<223>  n is a, c, g, or t

<220>
<221>  misc_feature
<222>  (285)..(285)
<223>  n is a, c, g, or t

<220>
<221>  misc_feature
<222>  (291)..(291)
<223>  n is a, c, g, or t

<220>
<221>  misc_feature
<222>  (294)..(294)
<223>  n is a, c, g, or t

<220>
<221>  misc_feature
<222>  (300)..(300)
<223>  n is a, c, g, or t

<220>
<221>  misc_feature
<222>  (303)..(303)
<223>  n is a, c, g, or t

<220>
<221>  misc_feature
<222>  (306)..(306)
<223>  n is a, c, g, or t

<220>
<221>  misc_feature
<222>  (315)..(315)
<223>  n is a, c, g, or t

<220>
<221>  misc_feature
<222>  (321)..(321)
<223>  n is a, c, g, or t

<220>
<221>  misc_feature
<222>  (330)..(330)
<223>  n is a, c, g, or t

<220>
<221>  misc_feature
<222>  (333)..(333)
<223>  n is a, c, g, or t

<220>
<221>  misc_feature
<222>  (336)..(336)
<223>  n is a, c, g, or t

<220>
<221>  misc_feature
<222>  (339)..(339)
<223>  n is a, c, g, or t

<220>
<221>  misc_feature
<222>  (345)..(345)
<223>  n is a, c, g, or t

<220>
<221>  misc_feature
<222>  (348)..(348)
<223>  n is a, c, g, or t

<220>
<221>  misc_feature
<222>  (354)..(354)
<223>  n is a, c, g, or t

<220>
<221>  misc_feature
<222>  (357)..(357)
<223>  n is a, c, g, or t

<220>
<221>  misc_feature
<222>  (360)..(360)
<223>  n is a, c, g, or t

<220>
<221>  misc_feature
<222>  (369)..(369)
<223>  n is a, c, g, or t

<220>
<221>  misc_feature
<222>  (375)..(375)
<223>  n is a, c, g, or t

<220>
<221>  misc_feature
<222>  (378)..(378)
<223>  n is a, c, g, or t

<220>
<221>  misc_feature
<222>  (381)..(381)
<223>  n is a, c, g, or t

<220>
<221>  misc_feature
<222>  (387)..(387)
<223>  n is a, c, g, or t

<220>
<221>  misc_feature
<222>  (393)..(393)
<223>  n is a, c, g, or t

<220>
<221>  misc_feature
<222>  (399)..(399)
<223>  n is a, c, g, or t

<220>
<221>  misc_feature
<222>  (405)..(405)
<223>  n is a, c, g, or t

<220>
<221>  misc_feature
<222>  (411)..(411)
<223>  n is a, c, g, or t

<220>
<221>  misc_feature
<222>  (417)..(417)
<223>  n is a, c, g, or t

<220>
<221>  misc_feature
<222>  (426)..(426)
<223>  n is a, c, g, or t

<220>
<221>  misc_feature
<222>  (429)..(429)
<223>  n is a, c, g, or t

<220>
<221>  misc_feature
<222>  (435)..(435)
<223>  n is a, c, g, or t

<220>
<221>  misc_feature
<222>  (438)..(438)
<223>  n is a, c, g, or t

<220>
<221>  misc_feature
<222>  (441)..(441)
<223>  n is a, c, g, or t

<220>
<221>  misc_feature
<222>  (444)..(444)
<223>  n is a, c, g, or t

<220>
<221>  misc_feature
<222>  (456)..(456)
<223>  n is a, c, g, or t

<220>
<221>  misc_feature
<222>  (462)..(462)
<223>  n is a, c, g, or t

<220>
<221>  misc_feature
<222>  (477)..(477)
<223>  n is a, c, g, or t

<220>
<221>  misc_feature
<222>  (480)..(480)
<223>  n is a, c, g, or t

<220>
<221>  misc_feature
<222>  (483)..(483)
<223>  n is a, c, g, or t

<220>
<221>  misc_feature
<222>  (498)..(498)
<223>  n is a, c, g, or t

<220>
<221>  misc_feature
<222>  (501)..(501)
<223>  n is a, c, g, or t

<220>
<221>  misc_feature
<222>  (504)..(504)
<223>  n is a, c, g, or t

<220>
<221>  misc_feature
<222>  (522)..(522)
<223>  n is a, c, g, or t

<220>
<221>  misc_feature
<222>  (540)..(540)
<223>  n is a, c, g, or t

<220>
<221>  misc_feature
<222>  (543)..(543)
<223>  n is a, c, g, or t

<220>
<221>  misc_feature
<222>  (546)..(546)
<223>  n is a, c, g, or t

<220>
<221>  misc_feature
<222>  (552)..(552)
<223>  n is a, c, g, or t

<220>
<221>  misc_feature
<222>  (558)..(558)
<223>  n is a, c, g, or t

<220>
<221>  misc_feature
<222>  (561)..(561)
<223>  n is a, c, g, or t

<220>
<221>  misc_feature
<222>  (567)..(567)
<223>  n is a, c, g, or t

<220>
<221>  misc_feature
<222>  (570)..(570)
<223>  n is a, c, g, or t

<220>
<221>  misc_feature
<222>  (585)..(585)
<223>  n is a, c, g, or t

<220>
<221>  misc_feature
<222>  (597)..(597)
<223>  n is a, c, g, or t

<220>
<221>  misc_feature
<222>  (609)..(609)
<223>  n is a, c, g, or t

<220>
<221>  misc_feature
<222>  (612)..(612)
<223>  n is a, c, g, or t

<220>
<221>  misc_feature
<222>  (621)..(621)
<223>  n is a, c, g, or t

<220>
<221>  misc_feature
<222>  (624)..(624)
<223>  n is a, c, g, or t

<220>
<221>  misc_feature
<222>  (627)..(627)
<223>  n is a, c, g, or t

<220>
<221>  misc_feature
<222>  (633)..(633)
<223>  n is a, c, g, or t

<220>
<221>  misc_feature
<222>  (636)..(636)
<223>  n is a, c, g, or t

<220>
<221>  misc_feature
<222>  (642)..(642)
<223>  n is a, c, g, or t

<220>
<221>  misc_feature
<222>  (648)..(648)
<223>  n is a, c, g, or t

<220>
<221>  misc_feature
<222>  (651)..(651)
<223>  n is a, c, g, or t

<220>
<221>  misc_feature
<222>  (657)..(657)
<223>  n is a, c, g, or t

<220>
<221>  misc_feature
<222>  (660)..(660)
<223>  n is a, c, g, or t

<220>
<221>  misc_feature
<222>  (663)..(663)
<223>  n is a, c, g, or t

<220>
<221>  misc_feature
<222>  (666)..(666)
<223>  n is a, c, g, or t

<220>
<221>  misc_feature
<222>  (669)..(669)
<223>  n is a, c, g, or t

<220>
<221>  misc_feature
<222>  (672)..(672)
<223>  n is a, c, g, or t

<220>
<221>  misc_feature
<222>  (678)..(678)
<223>  n is a, c, g, or t

<220>
<221>  misc_feature
<222>  (690)..(690)
<223>  n is a, c, g, or t

<400>  4
atgwsnwsnc arttyathtt ygargaygtn ccncarmgna aygcngcnac nttyaayccn      60

gargtnggnt aygtngcntt yathggnaar tayggncarc arytnaaytt yggngtngcn     120

mgngtnttyt tyytnaayca raaraargcn aaratggtny tncayaarac ngcncarccn 180

wsngtngayy tnacnttygg nggngtnaar ttyacngtng tnaayaayca yttyccncar 240

taygtnwsna ayccngtncc ngayaaygcn athacnytnc aymgnatgws nggntayytn 300

gcnmgntgga thgcngayac ntgyaargcn wsngtnytna arytngcnga rgcnwsngcn 360

carathgtna tgccnytngc ngargtnaar ggntgyacnt gggcngaygg ntayacnatg 420

tayytnggnt tygcnccngg ngcngaratg ttyytngayg cnttygaytt ytayccnytn 480

gtnathgara tgcaymgngt nytnaargay aayatggayg tnaayttyat gaaraargtn 540

ytnmgncarm gntayggnac natgacngcn gargartgga tgacncaraa rathacngar 600

athaargcng cnttyaayws ngtnggncar ytngcntggg cnaarwsngg nttywsnccn 660

gcngcnmgna cnttyytnca rcarttyggn athaayath 699

<210> 5
<211> 182
<212> PRT
<213> SBV Schmallenberg Virus cattle - nucleocapsid truncated

<400> 5

Met Val Leu His Lys Thr Ala Gln Pro Ser Val Asp Leu Thr Phe Gly
1               5                   10                  15

Gly Val Lys Phe Thr Val Val Asn Asn His Phe Pro Gln Tyr Val Ser
            20                  25                  30

Asn Pro Val Pro Asp Asn Ala Ile Thr Leu His Arg Met Ser Gly Tyr
        35                  40                  45

Leu Ala Arg Trp Ile Ala Asp Thr Cys Lys Ala Ser Val Leu Lys Leu
    50                  55                  60

Ala Glu Ala Ser Ala Gln Ile Val Met Pro Leu Ala Glu Val Lys Gly
65                  70                  75                  80

Cys Thr Trp Ala Asp Gly Tyr Thr Met Tyr Leu Gly Phe Ala Pro Gly
                85                  90                  95

Ala Glu Met Phe Leu Asp Ala Phe Asp Phe Tyr Pro Leu Val Ile Glu
            100                 105                 110

Met His Arg Val Leu Lys Asp Asn Met Asp Val Asn Phe Met Lys Lys
        115                 120                 125

Val Leu Arg Gln Arg Tyr Gly Thr Met Thr Ala Glu Glu Trp Met Thr
    130                 135                 140

Gln Lys Ile Thr Glu Ile Lys Ala Ala Phe Asn Ser Val Gly Gln Leu
145                 150                 155                 160

Ala Trp Ala Lys Ser Gly Phe Ser Pro Ala Ala Arg Thr Phe Leu Gln
                165                 170                 175

Gln Phe Gly Ile Asn Ile
            180

## Description

### TECHNICAL FIELD

The present invention relates to the cloning and expression of Schmallenberg virus (SBV) proteins or truncated proteins, their use in diagnostic tests to identify an Orthobunyavirus and in particular a SBV infection in a sample, and kits for their detection.

### BACKGROUND OF THE INVENTION

In 2011 cattle herds in various European countries showed an outbreak of a disease the origin and cause of which could not be linked to a known cause or disease at first.

The outbreak of this disease in European ruminants is characterized by fever, diarrhea and drop in milk yield in cattle as well as abortions, mummified fetuses, premature or stillbirths, and birth of weak or malformed lambs and calves (goat, sheep cattle).

Researchers of the Friedrich-Loeffler-Institut (FLI) were able to isolate a new virus not known so far in Europe. The isolate was identified in samples of a cattle herd in Schmallenberg, a small village in the Hochsauerland-Kreis in North Rhine-Westphalia, Germany. Accordingly, the virus was named Schmallenberg virus (SBV). The SBV was sequenced and the sequence data are accessible through the European Nucleotide Archive (https://www.ebi.ac.uk/ena/) under accession numbers HE649912, HE649913 and HE649914 (Hoffmann et al., 2012).

The virus is an enveloped, negative-sense, segmented, single-stranded RNA virus and composed of a S, M, L segment.

Other institutes isolated viruses from brain tissue of aborted lambs in France and the Netherlands.

SBV belongs to the Orthobunyavirus genus and Bunyaviridae family and it is related to the Simbu serogroup viruses (Shamonda, Akabane, Aino virus) (Hoffmann et al., 2012; Goller et al., 2012).

Orthobunyaviruses are well known pathogens for ruminants and humans in other areas of the world like Asia, Australia, Africa and South-America. They are spread by arthropods (vector borne disease).

SBV is most probably transferred by insects e.g. midges (Culicoides spec.).

Infected premises are found all over Europe (Germany, France, Belgium, The Netherlands, UK, Italy, Luxembourg, Switzerland, and Spain). The identified cases are most probably the result of infections that took place several months ago (gestation time for cattle app. 300 days, sheep and goats app. 150 days). The disease spreads out depending on the weather conditions and activity of the vector.

The SBV can be detected in a sample by way of RT-PCT. However, the viremic phase after virus infection is very short. Thus, viral genome sequences can only be detected in the peripheral blood for a couple of days. In addition, RT-PCT is very time consuming and requires a special laboratory and trained staff to perform the analysis. Thus this test cannot be considered an easy to use diagnostic test. Another disadvantage is that RT-PCT cannot provide a high throughput analysis either. Therefore, a high throughput test of an entire herd can only be performed with a big investment in financial resources and moreover it is very time consuming. Hence it cannot serve as screening tool in the context of SBV analysis and large animal herds.

Detection of antibodies directed against SBV proteins instead of viral nucleic acids has the advantage of using the immunological memory of infected animal. Antibodies are raised against viral proteins within 10 days after infection and they can be detected over a long period of time. The existence of specific anti-SBV antibodies is an indirect proof of a SBV infection.

Recently, an indirect ELISA using as antigen recombinant SBV nucleoprotein has been put on the market by ID-Vet (167, rue Mehdi Barka, F-34070 Montpellier) under the name "ID Screen® Schmallenberg virus indirect". The test is described for bovine, ovine, caprine. This test uses an indirect test format and recognizes only SBV for the indicted species.

Still there is a need to provide for a fast and reliable test for SBV infection screening world wide as an alternative and possibly with improved characteristics and applications.

Moreover, there exists the need to provide for a diagnostic test for the Simbu serogroup viruses and a pan-Simbu group test to be able to perform a fast and easy screening of herds for these infections. In view of the worldwide transport of animals a fast and reliable screening test is needed in order to make sure that infected animals are not transferred to non-infected herds. On the other hand in the course of purchasing animals the purchaser has an interest to buy healthy animals and not to learn only after having received the animals that they are infected with SBV or another Simbu serogroup virus. Such an infection will have an impact on the newborn calves and lambs and the final profit.

Hence, it is an object of the present invention to provide for a reliable SBV test, and alternatively to improve any known SBV test in order to provide for an improved and reliable SBV diagnostic test, preferably with high sensitivity and high selectivity.

It is yet another objection of the invention to provide for a validated diagnostic test for the detection of SBV.

It is yet another object of the invention to provide for a Simbu serogroup virus test and/or for a trans-species test.

### SUMMARY OF THE INVENTION

In one aspect the invention relates to the propagation and purification of SBV structural and non-structural proteins, and truncated versions of these proteins, in particular the nucleocapsid (N protein) of SBV.

In yet another aspect the invention relates to a method of identifying if a non-human animal is infected with a Simbu serogroup virus and particularly with SBV.

In yet another aspect the invention relates to a kit for the detection of a Simbu serogroup virus and particularly a SBV infection in a sample.

In yet another aspect the invention relates to a single step test format to identify SBV in a sample, preferably or alternatively to provide for a Simbu serogroup test, preferably in a single step test format as a Simbu serogroup test.

In yet another aspect the invention relates to an ELISA for the detection of the Simbu serogroup virus and preferably SBV infection in a sample.

In yet another aspect the invention relates to a validated diagnostic test for the detection of the Simbu serogroup virus and preferably SBV infection in a sample.

In yet another aspect the invention relates to an assay for the detection of Bunyaviruses of veterinary importance, in particular a Simbu serogroup assay.

In yet another aspect the invention relates to a kit for the detection of Bunyaviruses of veterinary importance, in particular of SBV and preferably of a Simbu serogroup virus.

### BRIEF DESCRIPTION OF THE FIGURES

**FIG. 1** depicts direct antigen (purified SBV nucleocapsid protein or nucleoprotein or N protein) coated to Maxisorp plates and the antigen titration in three different coating buffers tested with one positive, one negative and no sample.
**FIG. 2** depicts streptavidin pre-coated plates incubated with biotinylated N protein with decreasing protein amounts tested with three positive samples and one positive sample.
**FIG. 3** depicts the sequence of the nucleocapsid protein (N protein) of SBV (SEQ ID No. 1).
**FIG. 4** depicts the amino acid sequence of the nucleocapsid protein (HisN protein) of SBV (SEQ ID No. 2) including a His tag C-terminal, preferably used in a baculovirus expression system.
**FIG. 5** depicts the nucleocapsid protein with 6x His tag amino acid sequence according to Figure 4 wherein aminoacids (aa) in bold indicate important amino acids for Simbu group viral N protein.
**FIG. 6** depicts the SBV nucleocapsid protein full length with 6xHis tag DNA sequence in an pET28a vector (SEQ ID No. 3).
**FIG. 7** depicts Nucleocapsid protein DNA sequence with consensus codons (SEQ ID No. 4).
**FIG. 8** depicts Nucleocapsid truncated (protein-protein int. domain deleted): 182 aa His-tag C - terminal (SEQ ID No. 5).

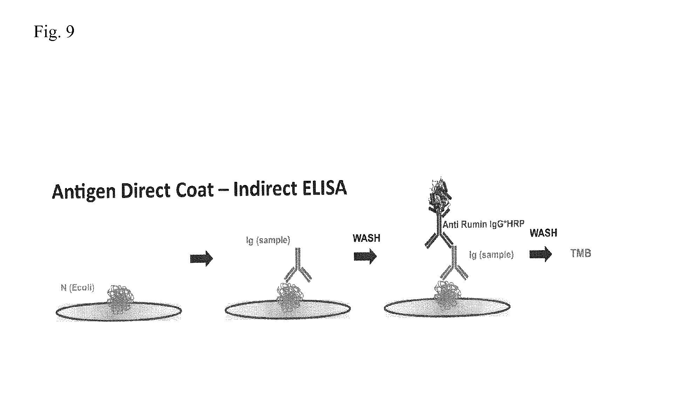

**FIG. 9** depicts an indirect ELISA format according to the invention.
**FIG. 10** depicts two single-step ELISA formats according to the invention; in the upper panel the antibody present in a positive sample is captured with one of the SBV virus proteins as described above. This protein is coated to a solid support. For detection a second anti-species specific or otherwise useful antibody is applied. This detection antibody is coupled with a detection means or can be visualized otherwise.

In the particular figure the SBV capturing protein is a nucleocapsid protein as depicted in the sequence listing.

The lower panel shows a different setup of the test wherein a SBV protein is used for capturing the anti-SBV antibody in the infected sample; this protein is coated to the solid phase; the detection is performed by a second SBV antigen which is bound by the second arm of the anti-SBV antibody in the sample.

In this second variation of the invention of a single-step ELISA an anti-ruminant-IgG-HRPO conjugate is replaced by a SBV nucleocapsid protein coupled to HRPO in a preferred emnbodiment. Thus an anti-SBV antibody of the sample of the infected animal can bind with one arm the one and with its other antibody arm the other SBV protein, and can thus be detected.

### DETAILED DESCRIPTION OF THE INVENTION

The various aspects of the invention will be described in the following in more detail.

The following definitions of important terms of the invention will apply throughout the specification, figures, examples and claims. Additional terms will be understood by the skilled person according to the general knowledge in the field.

As used herein, the singular forms "a," "an", and "the" include plural referents unless the context clearly dictates otherwise.

"SBV" as used herein refers to the Schmallenberg virus as isolated by the FLI (Friedrich-Loeffler-Institut) and having the sequences as described above. The SBV was sequenced and the sequence data are accessible through the European Nucleotide Archive under accession numbers HE649912, HE649913 and HE649914 (Hoffmann et al., 2012The virus is an enveloped, negative-sense, segmented, single-stranded RNA virus and composed of a S, M, L segment.

SBV belongs to the Orthobunyavirus genus and Bunyaviridae family and it is related to the Simbu serogroup viruses.

The "Simbu group viruses" or "Simbu serogroup viruses" or "pan-Simbu group viruses" as used herein comprises a group of Orthobunyaviruses which are serological related on the basis of cross-reactivity analyses (complement fixation, hemagglutination, and virus neutralization). Complement fixation activities are mediated by the viral nucleocapsid protein (N protein) while the viral glycoproteins are responsible for inducing neutralizing and hemagglutinating antibodies. Therefore a high degree of amino acid sequence homology in the N protein is one of the characteristics of the members of the Simbu serogroup. Some of the Simbu serogroup viruses are of veterinary importance and can be found in Asia, Australia, Africa, South-America and now in Europe as well. Although demarcation of Orthobunyavirus and Simbu serogroup viruses has proven difficult at least eighteen members of the serogroup including SBV are identified belonging to different virus species, e.g. the Simbu virus, Sathuperi virus, Douglas virus, Shamonda virus, Peaton virus, Sango virus, Akabane virus, Sabo virus, Tinaroo virus, Yaba-7 virus, Aino virus, Kaikalur virus, Shuni virus, Facey's Paddock virus, Oropuche virus, Utinga virus and Utive virus (Nichol et al., 2005).

"Amino acid" refers to naturally occurring and synthetic amino acids. Amino acid residues are abbreviated as follows: Alanine is A or Ala; Arginine is R or Arg; Asparagine is Nor Asn; Aspartic Acid is D or Asp; Cysteine is C or Cys; Glutamic Acid is E or Glu; Glutamine is Q or Gln; Glycine is G or Gly; Histidine is H or His; Isoleucine is I or Ile; Leucine is L or Leu; Lysine is K or Lys; Methionine is M or Met; Phenylalanine is For Phe; Proline is P or Pro; Serine is S or Ser; Threonine is T or Thr; Tryptophan is W or Trp; Tyrosine is Y or Tyr; and Valine is V or Val. Except where defined otherwise herein, X or Xaa represents any amino acid. Other relevant amino acids include, but are not limited to being, 4-hydroxyproline and 5-hydroxylysine. In all cases, the amino acid sequence of a polypeptide described or otherwise referred to herein is presented in conventional form in that the left-most, or first, amino acid residue of the sequence is the N- terminal residue and the right-most, or last, amino acid residue of the sequence is the C-terminal residue.

A "conservative variant" of any particular nucleic acid sequence includes any sequence having one or more degenerate codon substitutions to that particular nucleic acid sequence, any sequence having one or more nucleotide substitutions to, insertions to, and deletions from that particular nucleic acid sequence, and the complementary sequence of that particular nucleic acid and the conservative variants of that complementary sequence. Conservative variants of a particular nucleic acid sequence preferably have at least about 78% identity, more preferably have at least about 90% identity, and even more preferably at least about 95-99% identity, to that particular nucleic acid sequence. Conservative variants of a particular nucleic acid sequence may be artificially synthesized or they may be isolated in their natural form from an organism.

A "conservative variant" of any particular polypeptide sequence is any polypeptide having an amino acid sequence that varies from the amino acid sequence of that particular polypeptide but still retains the specific binding properties of that particular polypeptide, such that an antibody of the present invention that is raised against the particular polypeptide is capable of specifically binding the variant polypeptide. Therefore, for example, a conservative variant of a particular polypeptide may have one or more amino acid substitutions, deletions, additions, and insertions to that particular polypeptide. For example, a conserved variant of a particular polypeptide may have 30 or fewer, 25 or fewer, 20 or fewer, 15 or fewer, 10 or fewer, or 5 or fewer, conserved amino acid substitutions to that particular polypeptide. Conservative variants of a particular polypeptide preferably, but not essentially, have at least about 78% identity, more preferably have at least about 90% identity, and even more preferably at least about 91-99% identity, to that particular polypeptide. A percent identity for any subject nucleic acid or amino acid sequence (e.g., any of polypeptides described herein) relative to another "target" nucleic acid or amino acid sequence can be determined as follows. First, a target nucleic acid or amino acid sequence of the invention can be compared and aligned to a subject nucleic acid or amino acid sequence, using the BLAST 2 Sequences (B12seq) program from the stand-alone version of BLASTZ containing BLASTN and BLASTP (e.g., version 2.0.14). The stand-alone version of BLASTZ can be obtained at www.ncbi.nlm.nih.gov. Instructions explaining how to use BLASTZ, and specifically the B12seq program, can be found in the 'readme' file accompanying BLASTZ. The programms are also described in detail by Karlin et al. (1990) Proc. Natl. Acad. Sci. 87:2264;Karlin et al. (1990) Proc. Natl. Acad. Sci. 90:5873;and Altschul et al. (1997) Nucl. Acids Res. 25:3389.

As used herein, the term "polypeptide" refers to a compound of a single chain of amino acid residues linked by peptide bonds. The chain may be of any length. A protein is a polypeptide and the terms are used synonymously. The polypeptide is capable of binding one or more antibodies specific to different epitopes of the SBV polypeptides and preferably to recognize the Simbu group virus, more preferably to differentiate between the single members of said group of virus.

As used herein, the term "peptide" or "oligopeptide" refer to a single chain of amino acids consisting of between 3 and 25 amino acids, preferably of 5 to 12 amino acids. The proteins and polypeptides of the present invention are capable of eliciting an immune response in a host animal and/or are capable of binding an antibody present in a sample of an infected animal due to an immune response after infection.

Moreover, as part of the invention "antibody" or "antibodies" refers to monoclonal and/or polyclonal antibodies and anti-sera which can be applied in the methods, assays and test kits of the invention. These antibodies function as primary or secondary detection antibodies. These antibodies may be labeled for visualization of the test result. The antibodies are applied in concentrations known by the skilled person in the context of detection methods. Preferably the antibodies are applied in a concentration of 0.1 µg/ml -5.0 µg/ml

The antibodies applied in the present invention may belong to any antibody class, including for example, IgG, IgM, IgA, IgD and IgE, and may be prepared by any of a variety of techniques known to the skilled artisan. (See, e.g., Dean, Methods Mol. Biol. 80:23-37 (1998); Dean, Methods Mol. Biol. 32:361-79 (1994); Baileg, Methods Mol. Biol. 32:381-88 (1994); Gullick, Methods Mol. Biol. 32:389-99 (1994); Drenckhahn et al. Alethods Cell. Biol. 37:7-56 (1993); Morrison, Ann. Rev. Immunol. 10:239-65 (1992); Wright et al. Crit. Rev. Immunol. 12:125-68(1992); Harlow and Lane, Antibodies: A Laboratory Manual, Cold Spring Harbor Laboratory (1988); and Making and Using Antibodies: A Practical Handbook, Howard and Kaser, eds., CRC Press (2006), each one of which is incorporated herein by reference in its entirety.)

An "epitope" is the antigenic determinant on a polypeptide of SBV or the Simbu group virus or one virus of this group that is recognized for binding by a paratope on antibodies specific to said SBV polypeptide. Antibodies in the context of the invention recognize particular epitopes having a sequence of 3 to 11, preferably 5 to 7 amino acids. The antibody may further be characterized by its binding affinity to the protein, polypeptide or peptide applied in the methods and kits of the invention and the binding affinity is in the range of 10 ⁻⁹ to 10 ⁻¹⁰ nM Particular antibodies used in the invention are monoclonal or polyclonal antibodies directed against antibodies and applied as detection antibodies.

The term "label," as used herein, refers to a detectable compound, composition, or solid support, which can be conjugated directly or indirectly (e.g., via covalent or non- covalent means, alone or encapsulated) to a monoclonal antibody. The label may be detectable by itself (e.g., radioisotope labels, chemiluminescent dye, electrochemical labels, metal chelates, latex particles, or fluorescent labels) or, in the case of an enzymatic label, may catalyze chemical alteration of a substrate compound or composition which is detectable (e.g., enzymes such as horseradish peroxidase, alkaline phosphatase, and the like). The label employed in the current invention could be, but is not limited to alkaline phosphatase; glucose-6-phosphate dehydrogenase ("G6PDH"); horseradish peroxidase (HRP); chemiluminescers such as isoluminol, fluorescers such as fluorescein and rhodamine compounds; ribozymes; and dyes. The label may also be a specific binding molecule which itself may be detectable (e.g., biotin, avidin, streptavidin, digioxigenin, maltose, oligohistidine, 2, 4-dinitrobenzene, phenylarsenate, ssDNA, dsDNA, and the like). The utilization of a label produces a signal that may be detected by means such as detection of electromagnetic radiation or direct visualization, and that can optionally be measured.

A monoclonal antibody can be linked to a label using methods well known to those skilled in the art. E.g., Immunochemical Protocols; Methods in Molecular Biology, Vol. 295, edited by R. Bums (2005)). The detectable monoclonal antibody conjugate may be used in any known diagnostic test format like ELISA or a competitive assay format to generate a signal that is related to the presence or amount of an SBV-specific antibody in a test sample.

"Substantial binding" or "substantially bind" refer to an amount of specific binding or recognizing between molecules in an assay mixture under particular assay conditions. In its broadest aspect, substantial binding relates to the difference between a first molecule's incapability of binding or recognizing a second molecule, and the first molecules capability of binding or recognizing a third molecule, such that the difference is sufficient to allow a meaningful assay to be conducted to distinguish specific binding under a particular set of assay conditions, which includes the relative concentrations of the molecules, and the time and temperature of an incubation. In another aspect, one molecule is substantially incapable of binding or recognizing another molecule in a cross-reactivity sense where the first molecule exhibits a reactivity for a second molecule that is less than 25%, preferably less than 10%, more preferably less than 5% of the reactivity exhibited toward a third molecule under a particular set of assay conditions, which includes the relative concentration and incubation of the molecules. Specific binding can be tested using a number of widely known methods, e.g, an immunohistochemical assay, an enzyme-linked immunosorbent assay (ELISA), a radioimmunoassay (RIA), or a western blot assay.

A "biological sample" refers to a sample from an animal subject including saliva, whole blood, serum, plasma, milk or other sample known to contain anti-SBV antibodies. A biological sample can be collected and stored by any convenient means that does not impact the ability of the sample to be tested in the method of the invention. Samples for use in the method of the invention may require preparation prior to use in the method. For example, samples may need to be precipitated, diluted, filtered or centrifuged to remove unwanted components or components may be added to the sample such as salts, detergents or additional proteins.

The term "solid support" or "solid phase" refers to a non-aqueous matrix to which a SBV or a Simbu group virus polypeptide can adhere by covalent or non-covalent binding. Examples of solid support include supports formed partially or entirely of glass (e.g., controlled pore glass), synthetic and natural polymers, polysaccharides (e.g., agarose), polyacrylamides, polystyrene, polyvinyl alcohols and silicones, magnetic particles, latex particles, chromatographic strips, microtiter polystyrene plates, or any other substances that will allow bound antigens to be washed or separated from unbound materials. In certain embodiments, depending on the application, the solid support can be the well of an assay plate or can be a purification column (e.g., an affinity chromatography column).

As used herein, the term "kit" or "assay kits" (e.g., articles of manufacture) refers to an assembly of useful compounds and other means like solid support plates or test stripes for detecting SBV infection or Simbu group virus in a mammalian sample, preferably ruminants. A kit therefore may include one or more devices and/or compositions of the present invention. In one particular example, such a kit includes the device having an immobilized SBV protein, fragment thereof or peptide, and comprising one or more capture antibody and other useful reagents like wash reagent, as well as detector reagent and positive and negative control reagents, if desired or appropriate. Other components such as buffers, controls, and the like, known to those of ordinary skill in art, may be included in such test kits. The relative amounts of the various reagents can be varied, to provide for concentrations in solution of the reagents that substantially optimize the sensitivity of the assay. Particularly, the reagents can be provided as dry powders, usually lyophilized, which on dissolution will provide for a reagent solution having the appropriate concentrations for combining with a sample. The present kit may further include instructions for carrying out one or more methods of the present invention, including instructions for using any device and/or composition of the present invention that is included with the kit.

In the following the particular aspects of the invention will be described in more detail.

In one aspect of the invention SBV single proteins and polypeptides were expressed and purified. The full length and various truncated versions of the nucleocapsid (N) were cloned according to standard procedures known to the skilled person and produced in different expression systems. One such expression system used E.coli as is well known by the skilled person. Thus details need not be described herein and are well known from the literature. An alternative expression system that can be used is the baculovirus system also known in the art (Sambrook and Russell, 2001).

Particular details are also apparent from the Examples section.

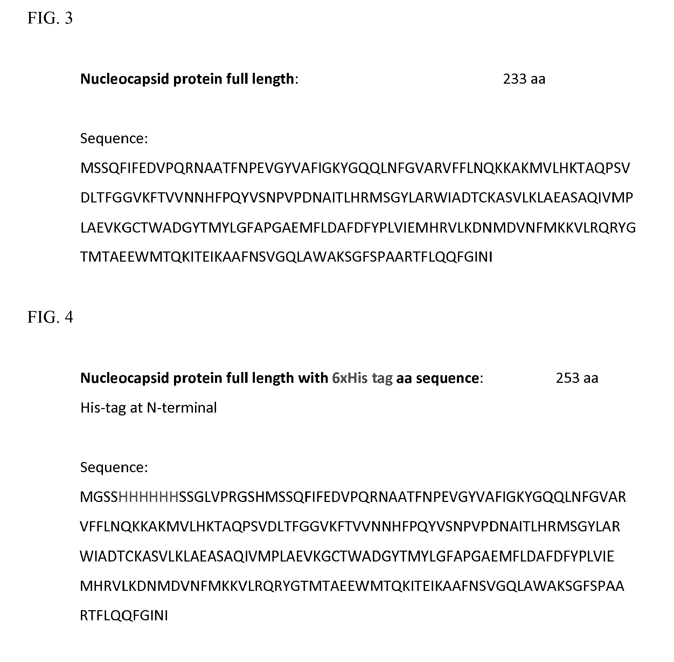

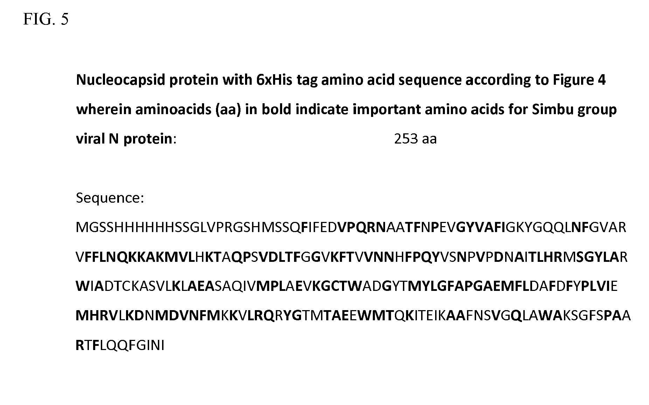

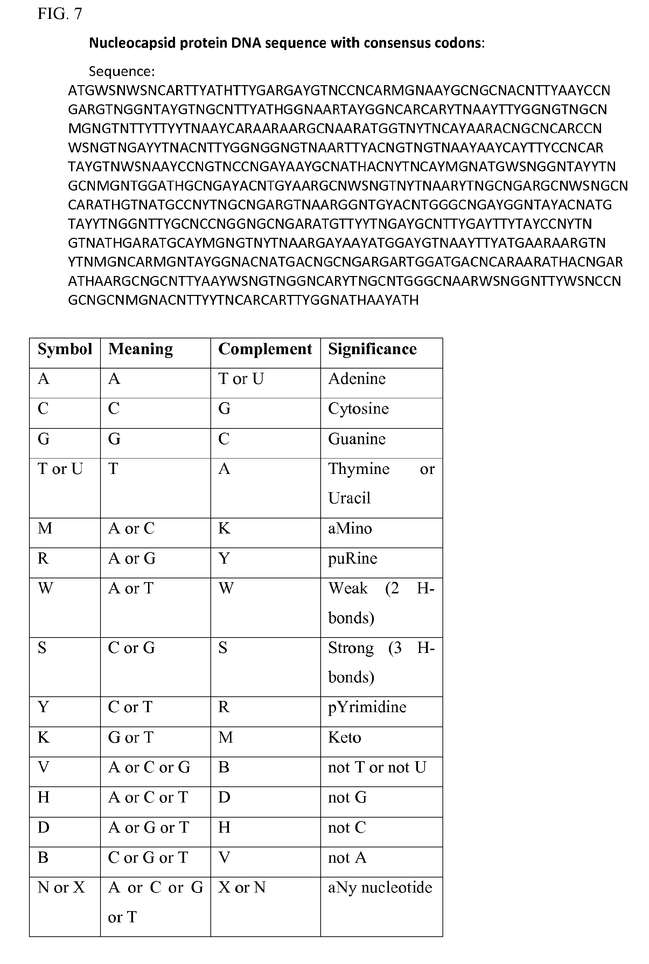

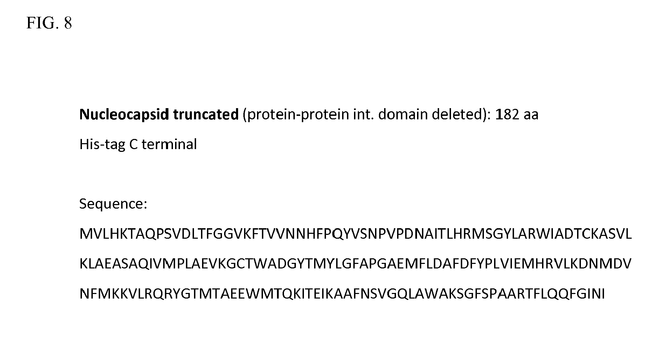

In one aspect the invention is an isolated SBV protein comprising or consisting of Nucleocapsid protein (N protein) having 233aa; a non-structural protein NSs (S segment) having 91 aa; a glycoprotein (Gc protein) having 846aa, preferably according to SEQ ID No. 1 to 5 (Figs. 3 to 8), or a fragment thereof, or a protein having 95% identity thereto for use in detection of SBV or a Simbu serogroup virus. In a preferred embodiment the isolated protein is an isolated SBV protein, polypeptide or peptide comprising or consisting of SEQ ID No. 1 to 5 or a peptide having 95% identity thereto.

Preferably the isolated peptide comprises or consists of an epitope useful for the specific identification of an SBV infection in a sample wherein the epitope comprises or consists of at least 3 amino acids, preferably at least 5 amino acids, more preferably at least 7 amino acids and even more preferably of 12 amino acids, even more preferably of between 5 and 25 amino acids of SEQ ID No. 1 to 5 (Figs. 3 to 8).

In another aspect the invention relates to a method of making a protein or polypetide as describe above.

The proteins and polypeptides of the invention can be cloned, expressed and purified according to methods well known in the art.

The use of different expression systems has advantages with regard to ease of the method and with regard to yield that can be achieved as well as secondary aspects like control of possible contaminations. If the proteins or peptides are applied in methods for diagnostic uses the production method may also have implications for their usefulness in the particular applications. In the current invention the SBV nucleocapsid (N) protein expressed in the E. coli expression system is preferred for diagnostic applications. While baculovirus expressed proteins can well be applied in diagnostic applications of the invention, E. coli expressed proteins are preferred.

The invention can use either a full length SBV N protein or truncated versions as well as such proteins which exhibit sequence modifications including but not limited to amino acid modifications, replacements, deletions, insertions or additions of sequences. Preferred embodiments are depicted in the figures and sequence listing. In a preferred embodiment the N protein as full length protein or including sequence modifications contains a C-terminal His-tag. In a preferred embodiment the N protein has a deletion of the protein-protein interaction domain and has more preferably an amino acid length of 180 to 200 amino acids, preferably the N truncated sequence has 182 amino acids. The particular sequences are depicted in the sequence listing and apparent from the Figures.

Particular SBV proteins and polypeptides can be expressed recombinantly and applied in standard diagnostic test formats well known to the skilled person. In the current invention an indirect ELISA format is particularly preferred, and more preferred is a single step ELISA format.

It could be shown that the SBV proteins as described above are particularly useful in tests and methods according to the invention to detect preferably SBV infection, and in a more preferred embodiment Simbu serogroup viruses.

Particularly useful is the full length SBV N protein as of SEQ ID No. 1. More preferred is a truncated N protein as depicted in SEQ ID No. 5.

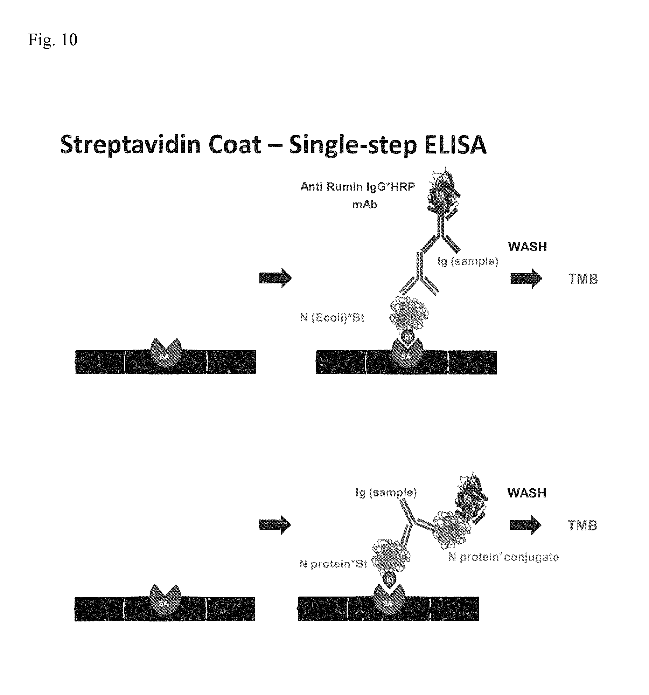

It could be shown that the application of said protein is showing particularly good results with regard to specificity and selectivity. While all known detection formats can in principal be applied for the test, certain test formats will be preferred in view of practical aspects like ease of performing them and provision of test kits. In a preferred embodiment advantageously good results were achieved in an indirect ELISA format as depicted in the Figures. More preferred is a single-step ELISA which has the advantage that all reagents and the sample can be mixed in a one-step-process. Various preferred embodiments are depicted in Fig. 10.

Further details as to the sequence and production of the proteins are depicted in the Figures and the sequence listing.

In a preferred embodiment the invention relates to a method of identifying a non-human animal infected with SBV or a Simbu serogroup virus comprising the steps: i. contacting a sample with a protein, polypeptide, oligopeptide or peptide of SBV under conditions that a complex of an antibody specific against SBV in the sample and the protein, polypeptide, oligopeptide or peptide of SBV can form; ii. detecting the thus formed complex with an antibody specific against the antibody of the complex wherein the detection of the complex is indicative of an infection with SBV.

In a preferred embodiment the method of identifying is an ELISA (enzyme linked immuno sorbent assay), preferably a multi-step and more preferably a single-step method.

In such a method a protein, polypeptide, oligopeptide or peptide of SBV can be applied and preferably it is selected from the group consisting of SEQ ID No. 1 to 5 (Figs. 3 to 8), or a fragment thereof.

In another aspect the invention relates to a kit for the detection of a SBV or Simbu serogroup virus infection in a sample comprising i. one or more proteins, polypeptides, oligopeptides or peptides of SBV as described in the Figures and sequence listing; and ii. a means for detection of a complex formed between one or more of the protein, polypeptide, oligopeptide or peptide of SBV and an antibody specific against SBV.

The protein, polypeptide, oligopeptide or peptide of SBV is preferably selected from the group consisting of SEQ ID No. 1 to 5 (Figs. 3 to 8).

In another aspect the invention is a method for detecting antibodies to SBV in a biological sample, the method comprising contacting the biological sample with a SBV polypeptide of SEQ ID No. 1 to 5, and detecting whether antibodies in the sample compete with the monoclonal antibody for binding to SEQ ID No. 1 to 5, thereby determining that the sample contains antibodies to SBV, wherein the SBV antibodies in a sample from animals that have been naturally infected will compete with the monoclonal antibody for binding to SEQ ID No. 1 to 5 but antibodies in a sample from animals that have not been infected or animals that have been vaccinated against SBV more than 4 weeks prior to the sample being obtained will not compete with the monoclonal antibody for binding to SEQ ID No. 1 to 5. In a preferred embodiment the method can be used for the detection of Simbu group viruses.

The invention achieves a sensitivity of more than 95%, preferably more than 96%, even more preferred more than 97% and a specificity of more than 97%, preferably more than 98%, and even more preferably more than 99% with the truncated N protein according to SEQ ID No. 1 to 5.

The samples that can be used in the methods, assays and test kits of the invention are from serum, plasma or milk preferably of ruminants and are particularly useful to detect infection in cattle, sheep or goat.

The kit may contain ready to use reagents and the test results are advantageously obtained within several hours, preferably within less than 6 hours, preferably less than 5 more preferably less than 4 hours.

The sample dilution factor is preferably less than or equal 50x, preferably 30x, preferably 20x, preferably 10x which advantageously avoids further dilution steps.

The kit preferably contains all ready to use reagents including coated plates, negative and positive controls, wash solution, sample diluent, conjugate, TMB and stop solutions.

The antigen applied is preferably an antigen which is conserved among the Simbu group viruses and thus advantageously allows a pan-Simbu group detection of infection in a sample.

In preferred embodiments the solid phase of the test is coated with antigen as described above. The antigen can be expressed in E.coli. Particularly preferred is an antigen expressed in a Baculovirus expression system. In the method and test kit any known and useful solid phase may be used, preferably MaxiSorp or PolySorp (Thermo Fisher Scientific) is used and coated by applying a coating buffer which has preferably a pH of 5, 7 pr 9.5. The antigen is applied in a quantity of 1, 2, 3 or 4 ug/ml. It may be useful to apply a blocking solution known in the art but it is preferred to avoid a blocking solution.

As sample diluent may be used 0.14M NaCl, 2.7mM KCl, Kathon 0.03%, Tween20 0.1 %. Another diluent useful according to the invention is 2% MgCl2, 6% Tween20 and 6% AO, 0.5% Casein sodium salt

The detection antibody is diluted 1:10000, preferably 1:20000.

The method steps will be applied as required and may vary depending to the particular reagents applied. In a preferred embodiment the conditions and method steps are as follows:
Sample (1:10) in sample diluent (MgCl2 2%, AO 6%, Tween20 6%, Casein 0.5%), 100 ul/well
Incubate 1 h, room temperature in humid chamber
3x wash (phosphate buffered saline with 0.1% Tween20)
Conjugate ready-to-use, 100 µl/well
Incubate 1 h, room temperature in humid chamber
3x wash (phosphate buffered saline with 0.1% Tween20)
TMB 100ul/well, incubate 10 mns, room temperature
Add stop solution (100 µl/well) and
Read out at 450nm

In particularly preferred embodiments the sample diluent contains casein sodium salt in a concentration of between 0.1 to 0.55 %, preferably 0.1 %, more preferably 0.55 %. In preferred embodiments the sample diluent contains 0.5 % casein sodium salt and MgCl2, preferably the MgCl2 has a concentration of 2%.

In a preferred embodiment the method of detection, and preferably also the kit, is characterized by the inclusion of specific compounds, the use of particular dilutions of the capturing antigen and/or a particular amount and quality of capturing antigen coated onto the solid support used in the method of detection and the kit of the invention.

It could be shown that by way of the particular combination of preferably the purification conditions and/or the dilution conditions and/or the particular components included in the method of detection and kit an improved performance of the method of SBV detection and preferably of Simbu serogroup viruses could be achieved.

All members of this group could be detected with the method of the invention. Certain viruses are preferably detected: Schmallenberg virus, Shamonda virus, Douglas virus, Peaton virus, Akabane virus, Aino virus, Oropouche virus, Tinaroo virus, Shuni virus. Particularly preferred and particular good results were achieved with the following species: Peaton, Acabane, Aino, Tinaroo. In a particularly preferred embodiment the Simbu serogroup viruses of Bovidae, Cervidae and Camelidae are detected.

In a particularly preferred embodiment the dilution of the SBV protein, preferably the nucleocapsid protein, was chosen to be in the coating solution in a concentration of 0.5 to 5 µg/ml, preferably 1 to 3 µg/ml. The solid support to which the SBV protein is coated contains antigen from 0.25 to 1µg/well, preferably 0.5 to 1µg/well. The inventive nucleocapsid preparation in its particular dilution proofed advantageous.

Accordingly, it was achieved to have the SBV coating protein substantially in solution and the formation or presence of aggregates of the coating protein, preferably the nucleocapsid protein as described above and in SEQ ID No. 1 to 5, could be avoided. Hence, the specificity of the test could be increased and it was not evident that this aspect of the test could lead to the advantageous specificity and/or sensitivity. The positive results are evident from performing the test with confirmed positive and negative samples regarding SBV infection as such.

Moreover, the advantage of the test and kit according to the invention as described herein is not only evident from the positive test results achievable with the test as such. It is more so evident in comparison with test kits on the market as of today, i.e. September 2012. In particular the current invention and the method of detection in preferred embodiments is superior to SBV tests of the prior art, like the IDvet test and kit described above in the background section.

Another preferred embodiment uses in the method of detection and preferably in the kit is casein. Any casein available in the market can be applied in a concentration which can be adapted according to the needs of the other compounds and in order to achieve good detection results in the test. Preferably the concentration in a solution of the test is 0.1 to 1 %, preferably 0.25 to 0.75 %, more preferably 0.5 %. In a preferred embodiment the casein is present in the wash solution of the coating step wherein the antigen is coated to the solid support device.

The coating step is preferably performed at pH 5 to 10, preferably about 5, 7 or 9.5.

The coating protein, i.e. the antigen which may be preferably SBV nucleocapsid protein as described in the specification and Examples, is used in amounts of 1, 2, or 4 µg/ml, preferably 1 µg/ml.

Another preferred embodiment uses alone or in addition aminoxid (AO). Preferably the concentration used is in a solution of the method in an amount of 3 to 10%, preferably 5 to 8%, more preferably 6%.

Another preferred embodiment of the method of detection and kit contains Tween, preferably a Tween20, or a comparable substance, preferably a detergent with comparable characteristics. This substance is contained in an amount of 0.05 to 0.5%, preferably 0.1 to 0.2 %.

In a particularly preferred embodiment the wash solution of the coating step contains preferably NaCl 0.14M, KCl 2.7 mM; Kathon 0.03%, Tween20 0.1%, sample diluent comprises MgCl2 2%, aminoxid (AO) 6%, Tween20 6% and 0.5% casein.

In a preferred embodiment, the anti-ruminant conjugate is used in a dilution of 1:10 000 to 1:30 000, preferably 1:20 000 in a conjugate stabilizing buffer as a ready to use format (RTU).

In another preferred embodiment the above described compounds can be combined and will lead to particularly good test results. In such an embodiment the various compounds are contained in any of the solutions used for preparation of the kit compounds or during performing the test and may be contained in the kit according to the invention.

The inventors surprisingly achieved with one or a combination of the above described preferred embodiments very good results with regard to specificity and selectivity. It was not foreseeable that such good results could be obtained by using the particular antigen preparation in its particular dilution is particularly advantageous. More advantageous is the antigen dilution in connection with the other additives like casein, aminooxid and/or Tween, preferably in the concentrations as described above.

The test preferably detects the Simbu serogroup viruses, it can preferably detect SBV in bovine, goat and sheep samples.

The cut off can be chosen according to the needs of sensitivity and specificity.
Distribution is regarded to adapt it to fitness for purpose. This will depend on the needs of the test. ROC is used for analysis. The cut off is chosen according to standard procedures with regard to a positive and negative controls and the respective calculations known to the person skilled in the art and literature for disgnostic test development.

In order to optimize the specificity the cut off is chosen preferably at 30% or more, 40% or more, 50% or more, or 60% or more.

In order to optimize the sensitivity the cut off is chosen preferably at 10% or more, 20% or more, 30% or more, or 40% or more.

An optimal cut off for the test according to the invention is preferably 40%, more preferably between 30 and 40%.

The advantage of the tests of the invention is that in preferred embodiments a specificity of more than 97%, preferably 98% and more preferably more than 99%, and a sensitivity of more than 94%, more preferably more than 95%, and more preferably more than 96% can be achieved. Particular embodiments achieve a specificity of about 99.8% and a sensitivity of about 95.8%. With regard to particular species the following specificities and sensitivities, respectively, can be achieved: cattle: 99.5% and 95.7%; sheep: 100% and 99%; goat: 100% and 99%.

Accordingly, the method and test kit will be particularly reliable for goat, sheep and cattle for the detection of SBV. In a preferred embodiment the good results will also apply to the Simbu serogroup. In particular good results can be achieved to detect Akabane, Peaton, Aino and Tinaroo virus.

In the following the invention will be illustrated by way of preferred examples which are not meant to be restrictive in any sense.

### EXAMPLES

### Example 1

Development of a first generation screening test for detection of antibodies directed against Schmallenberg virus (Simbu serogroup ELISA)

In a first step the sequences of various Bunyaviruses were compared which is apparent from Table 1 below. Members of the Simbu serogroup showed a sequence similarity to the N protein of SBV of at least 78%.

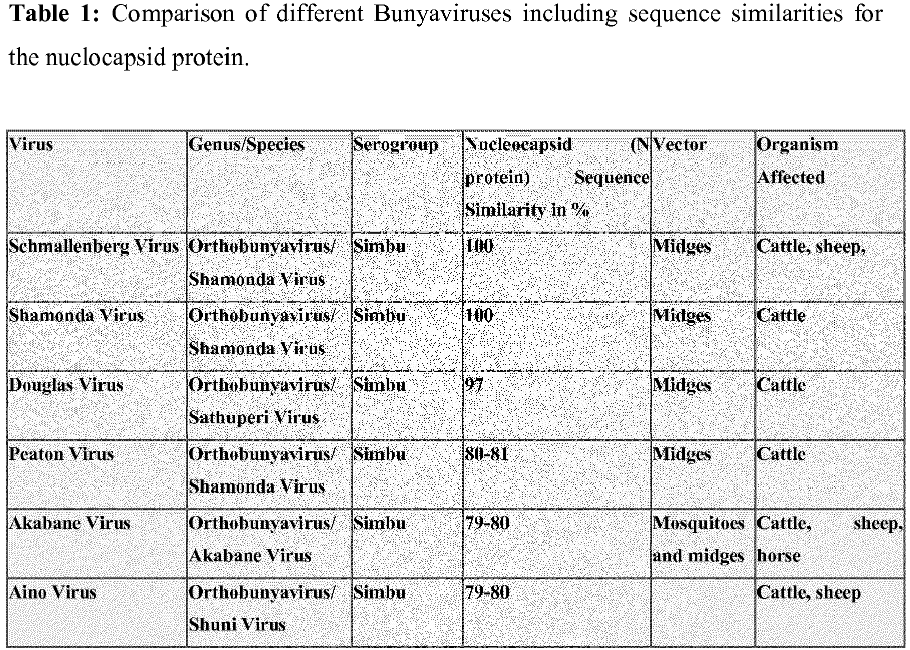

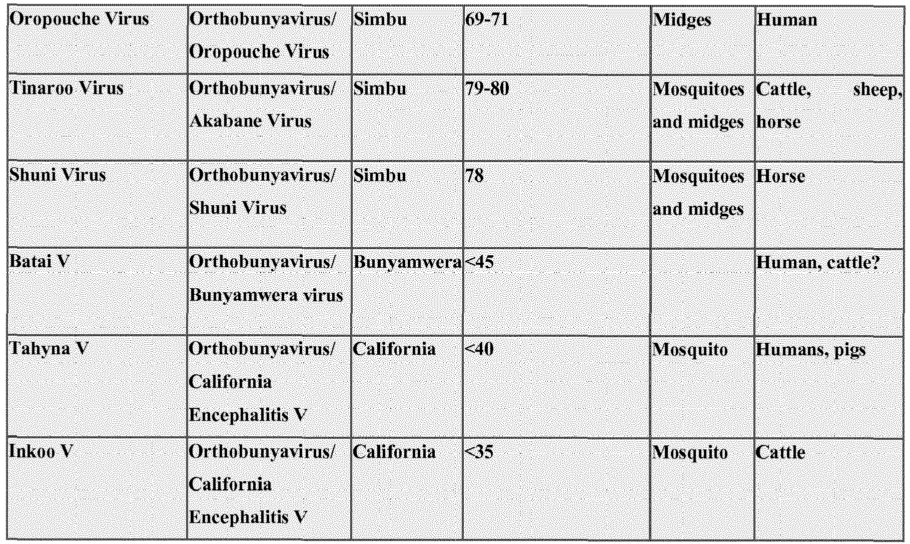

The sequence of the N protein clone is identical to the sequence SBV/1-846 presented in Table 2 below. The clone contains an additional His-tag.

### Expression, purification and biotinylation

The plasmid DNA was use to transform bacterial host cells and express the viral N (nucleocapsid) protein. A standard affinity purification system was established using Ni columns (His tagged N protein). The N protein shows a strong tendency to precipitate in buffered solutions, but only the buffer soluble fraction was used for further purification The binding and wash buffers contained 10 mM imidazole to prevent unspecific binding of contaminating E. coli proteins to the Ni column. The N protein was released from the column by increasing the imidazol concentration to 0.5 M in the elution buffer. Eluted proteins were used for direct coating experiments after removal of imidazol by dialysis against carbonate buffer pH 9.

Biotinylation of the N protein was done in alkaline carbonate buffer. Activated biotin was added and bound to the N protein. The biotinylation process was stopped by adding Tris buffer and free biotin was removed by dialysis against carbonate buffer without biotin. This material was used for coating experiments using plates pre-coated with streptavidin (plates from IDEXX CoE in Westbrook, ME, USA).

### Direct coating of protein to the plates:

The purified N protein (not biotinylated) was coated on Maxisorp plates using standard procedures (phosphate buffer, overnight incubation at 4°C). Plates were dried and antibody positive and negative samples were tested.

Coating Protocol:
- Dilution of purified N protein in phosphate buffer pH 7
- Addition of detergent to 15 µg/mlfinal concentration
- Incubation at room temperature for 20 min with shaking
- Further dilution of detergent treated antigen to 1 - 4 µg/ml final concentration in phosphate buffer
- Coating of Maxisorp plates with 100 µl/ well
- Incubation over night at 4°C in a humid chamber
- Aspiration of coating solution and drying of plates

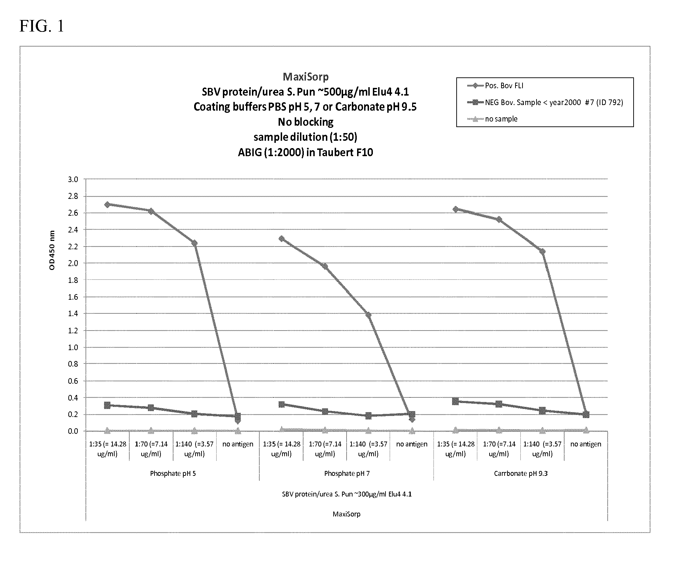

FIG. 1 depicts the results of the experimentation and is an example of antigen (purified N protein not biotinylated) titration in three different coating buffers (PBS pH 5; PBS pH 7; sodium carbonate buffer pH 9.3) tested with one positive (Pos. Bov FLI), one negative (NEG Bov. Sample) and no sample/blank.

### Example 2

### Streptavidin pre-coated plates and coupling of biotinylated N protein to the plates

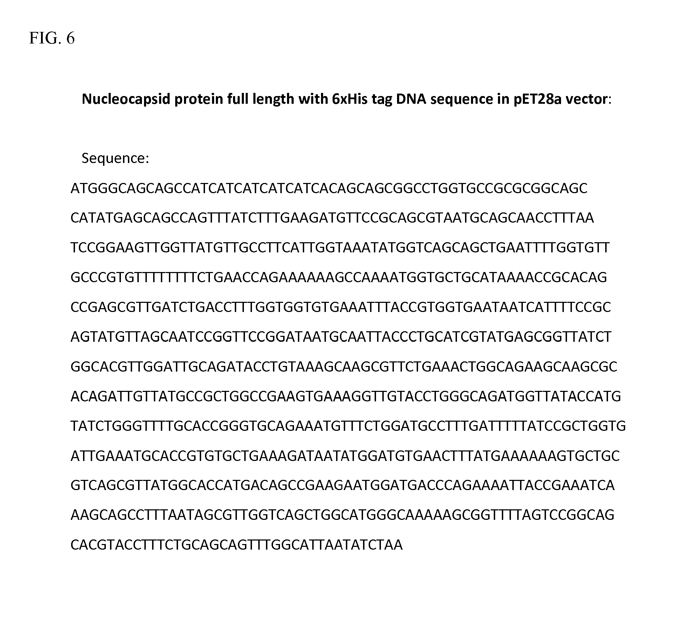

Plates were pre-coated with streptavidin. Streptavidin plates were incubated with biotinylated N protein overnight, washed three times, and were not dried before the ELISA protocol was run. FIG. 6 shows an example of N protein biotinylated using a molar ratio of 2:1 (biotin:protein). Three positive samples were tested and one negative sample. The negative sample (ID 792) was used in the direct coat experiments as well (cf. Example 1). The positive samples are different from those in example 1. The ELISA protocol is identical with the protocol described for direct coat above of example 1.

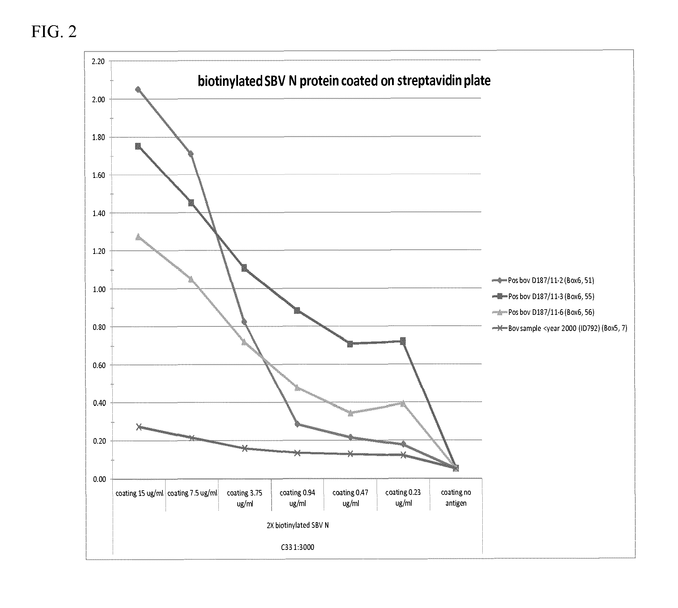

FIG. 2 shows that in both cases there is a good differentiation between positive and negative samples over a range of various antigen concentrations.

### Example 3

### ELISA format I

In this format a direct coating of recombinant SBV nucleocapsid (N) protein is used. The coating protein can be either full length nucleocapsid protein or a truncated version according to SEQ ID No. 1 to 5. Other SBV proteins as depicted in the sequence listing can also be applied in the test.

The format is indirect and monophasic, and the sample type can be serum, plasma or milk.

The coated plates are incubated with serum, plasma or milk at room temperature. The samples can be applied as individual samples in single wells, in a dilution series and preferably as duplicates or triplicates. The sample dilution is preferably 1:10 or 1:20. After incubation for about 10 to 90 minutes, preferably about 30 to 60 minutes the plates are 3x washed preferably with PBS. For detection an anti-ruminant HRP-conjugated antibody is applied to the plate and incubated for about 10 to 90 minutes, preferably about 30 to 60 minutes. After a 3x wash substrate, preferably TMB is added for about 5 to 15 minutes, preferably 10 minutes, and the result is read out at 450nm. The plate is set up to include wells for samples and as well for positive and negative controls.

The recombinant nucleocapsid protein can be produced in E. coli or in a baculovirus system. Details as to the nucleocapsid protein used according to the invention are depicted in the sequence listing. Any of the nucleocapsid proteins listed therein can be applied in the test.

The coating is performed at a pH of about 5, 7 or 9.5 with or without blocking, preferably at pH 7. The coating protein is applied in amounts of o, 1, 2, or 4 µg/ml, preferably 1 µg/ml.

The wash solution contains preferably NaCl 0.14M, KCl 2.7 mM; Kathon 0.03%, Tween20 0.1%, sample diluent comprises MgCl2 2%, aminoxid (AO) 6%, Tween20 6% and 0.5% casein.

The anti-ruminant conjugate is used in a dilution of 1:10 000 to 1:30 000, preferably 1:20 000 in a conjugate stabilizing buffer as a ready to use format (RTU).

The test can detect SBV in bovine, goat and sheep samples.

### Example 4

### ELISA format II - single step ELISA

The recombinant SBV protein, preferably nucleocapsid protein as described under ELISA format I is coupled to the solid support via streptavidin - biotin. Preferably the streptavidin is coupled to the solid support and the SBV protein is coupled with biotin thus allowing coating to the solid support via the streptavidin-biotin complex that forms.

The solid support, e.g. a multiwall plate, is coated with streptavidin. The streptavidin coated plates are incubated in one single step with the biotinylated SBV protein (as described above; reference is made to the sequence listing of SBV proteins herein), the sample (preferably diluted as described above) and the anti-ruminant monoclonal antibody conjugated with HRP. The HRP antibody is an IgG preferably. After incubation for about 30 to 90 minutes, preferably 1 hour the plate is washed as described above and the read out is done at 450nm.

Microtiter plates are supplied precoated with streptavidin. The all-in-one buffer contains the biotinylated nucleocapsid protein, and the anti-IgG conjugate. Dilutions of the samples to be tested are incubated in the all-in-one buffer in wells of these plates. Any antibody specific for SBV binds to the antigen in the all-in-one buffer and forms an antigen/antibody/anti-IgG conjugate complex on the streptavidin-plate well surface. Unbound material is removed from the wells by washing. The TMB containing substrate is added to the wells. The degree of color that develops (optical density measured at 450 nm), is directly proportional to the amount of antibody specific for SBV present in the sample.

Results of the single step ELISA according to the invention in comparison to the IDvet test of the prior art are illustrated in Table 5

### Example 5

### Simbu serogroup recognition by ELISA (pan-Simbu group ELISA)

Preferred test formats according to the invention are capable of recognizing antibodies formed after infection with different members of the Simbu serogroup, i.e. the test is a pan-Simbu group test. See also Table 6.

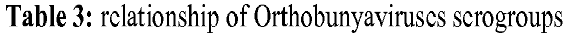

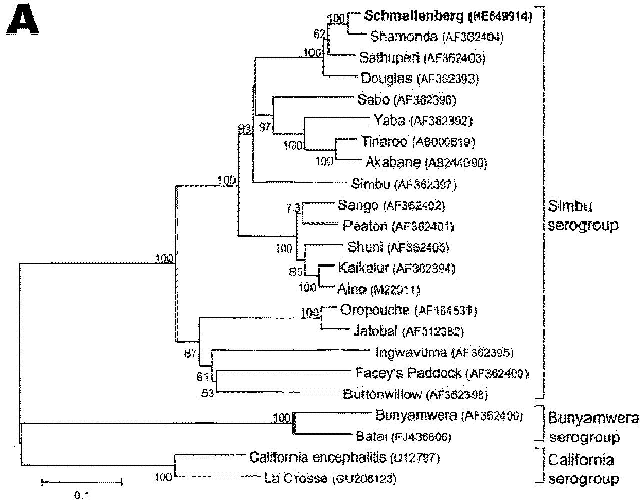

In a preferred embodiment the test is capable to detect the viruses of the Simbu serogroup, in a further preferred embodiment the Simbu and Bunyamwera, in a further preferred embodiment in addition the California serogroup, alternatively the Simbu and California serogroup in one test setup.

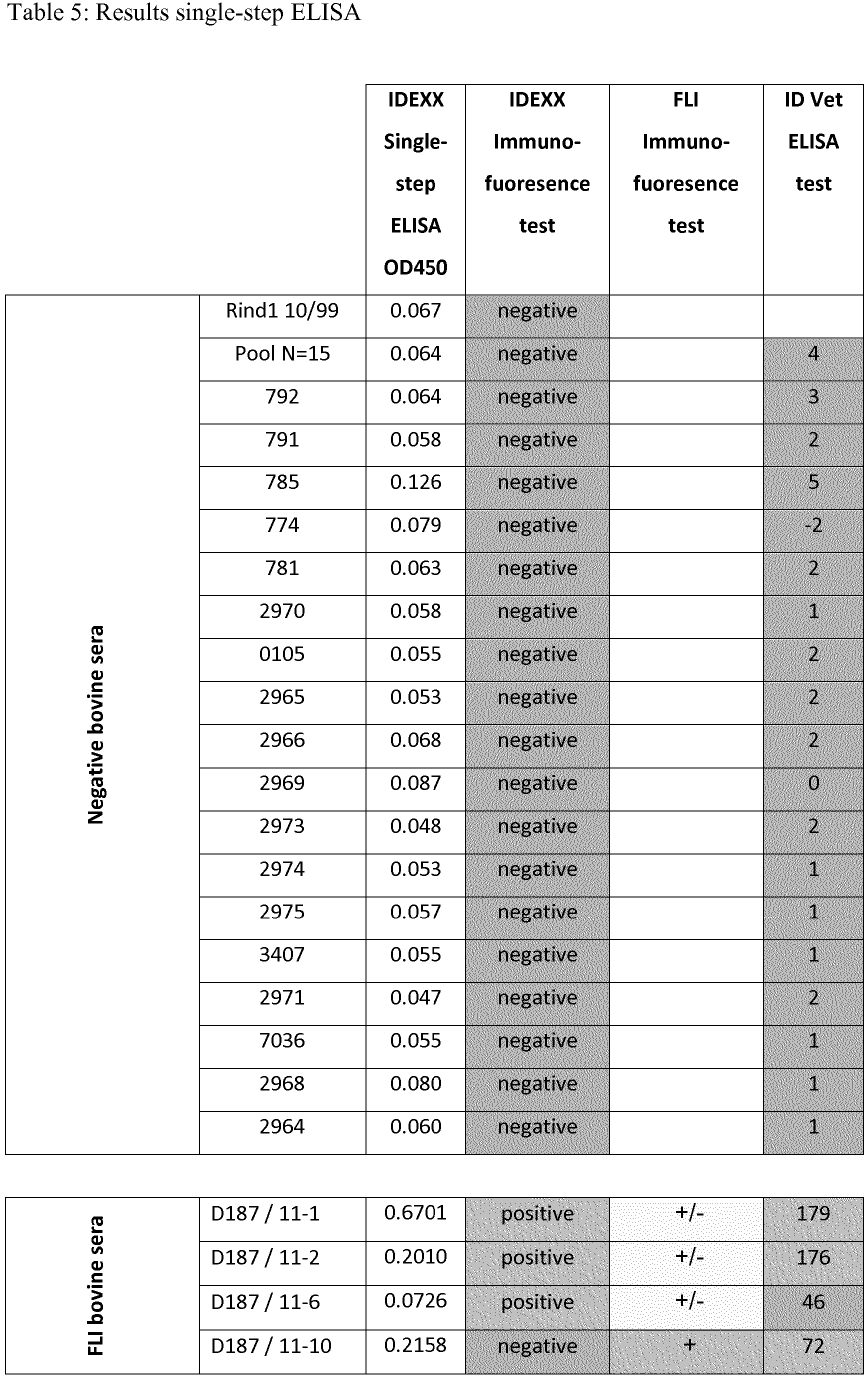

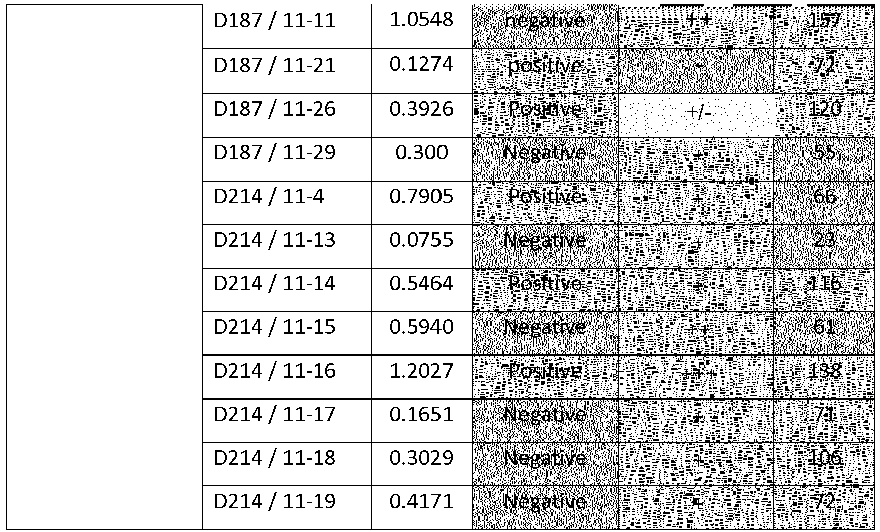

Table 5 describes a comparison of validated samples in the test according to the invention vis-à-vis the test marketed by IDvet.

The test results show the superior performance of the test and method according to the invention.

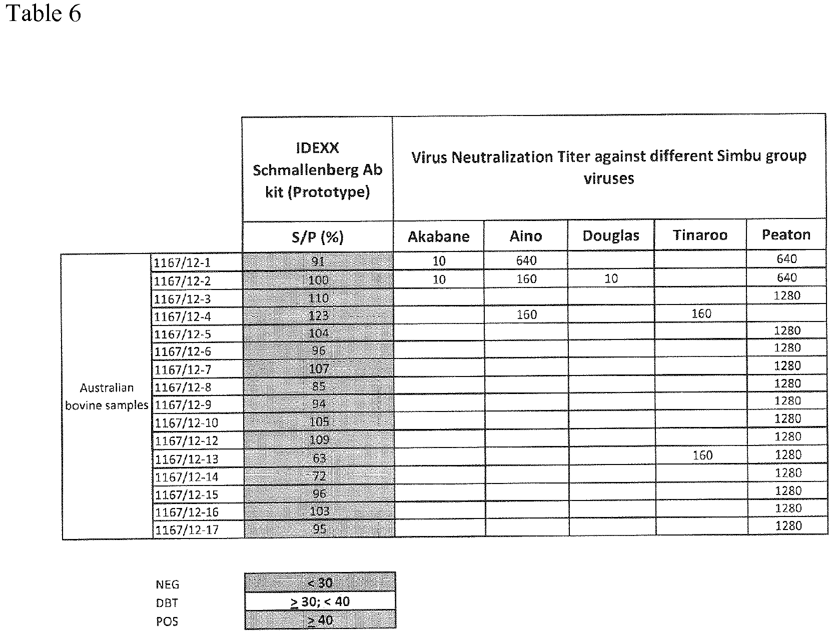

Table 6 shows validated bovine samples used in the test according to the invention. It confirms the detection of various Simbu serogroup viruses and thus confirms its usefulness as pan Simbu serogroup test.

### LITERATURE LIST

Hoffmann, B., Scheuch, M., Höper, D., Jungblut, R., Holsteg, M., Schirrmeier, H. (2012). Novel Orthobunyavirus in cattle, Europe, 2011. Emerg. Infect. Dis. 18, 469-472
Saeed, M. F., Li, L., Wang, H., Weaver, S. C., and Barret, A. D. T. (2001). Phylogeny of the Simbu serogroup of the genus Bunyavirus. J. Gen. Virol. 82, 2173-2181.
Goller, V. K., Höper, D., Schirrmeier, H., Mettenleiter, T. C., and Beer, M. (2012). Schmallenberg Virus as Possible Ancestor of Shamonda Virus. Emerg. Infect. Dis. 18,
Nichol, S. T., Beaty, B. J., Elliot, R. M., Goldbach, R., Plyusnin, A., Schmaljohn, C. S., and Tesh, R. B. (2005). Family Bunyaviridae. Edited by C. M. Fauquet, J. A. Mayo, J. Maniloff, U. Desselberger & L. A. Ball. Virus Taxonomy: Eighth Report of the International Committee on Taxonomy of Viruses. San Diego, CA, Elsevier, pp. 695-716.
Sambrook, J. and Russel, D. W. (2001). Molecular cloning: a laboratory manual. Third Edition, Cold Spring Habor Laboratory Press, Cold Spring Habor, New York
Eifant, S. A., Elliott, R. (2009). Mutational analysis of the Bunyamwera Orthobunyavirus nucleocapsid protein gene. J. Virol. 83, 11307-11317.

## Claims

1. An isolated SBV protein comprising a nucleocapsid protein 233aa, preferably according to SEQ ID No. 1 to 5 (Figs. 3 to 8), or a fragment thereof, or a protein having 95% identity thereto for use in a Schmallenberg virus (SBV) detection assay or a Simbu serogroup assay.

2. An isolated peptide comprising an epitope useful for the specific identification of antibodies to SBV in a sample, preferably to Simbu serogroup virus wherein the epitope comprises at least 5 amino acids, preferably at least 7 amino acids and more preferably of 12 amino acids, even more preferably of between 5 and 25 amino acids of SEQ ID No. 1 to 5 (Figs. 3 to 8),

3. A method of making a protein according to claim 1 or a peptide according to claim 2.

4. A method of identifying a non-human animal infected with SBV or a Simbu serogroup virus comprising the steps: i. contacting a sample with a protein, polypeptide, oligopeptide or peptide of SBV under conditions that a complex of an antibody specific against SBV or against a Simbu serogroup virus in the sample and the protein, polypeptide, oligopeptide or peptide of SBV of SEQ ID No. 1 to 5 (Figs. 3 to 8) can form; ii. detecting the thus formed complex with an antibody specific against the antibody of the complex wherein the detection of the complex is indicative of an infection with SBV or a Simbu serogroup virus.

5. Method according to claim 4 wherein the method is an ELISA (enzyme linked immuno sorbent assay), preferably a multi-step or single-step method.

6. A kit for the detection of a SBV infection or a Simbu serogroup virus in a sample comprising i. a protein, polypeptide, oligopeptide or peptide of SBV SEQ ID No. 1 to 5 (Figs. 3 to 8); and ii. a means for detection of a complex formed between the protein, polypeptide, oligopeptide or peptide of SBV and an antibody specific against SBV or a Simbu serogroup virus.
